# FASCICULE DE BREVET EUROPEEN

(11) **EP 3 265 332 B1**
(45) Date de publication et mention de la délivrance du brevet: **19.01.2022**
(21) Numéro de dépôt: 16701130.3
(22) Date de dépôt: 20.01.2016
(51) Int. Cl.: B60H 3/00, A61L 9/12

(54) **SYSTÈME D'IMMOBILISATION D'UNE CARTOUCHE AMOVIBLE DANS UN BOÎTIER**
SYSTEM ZUR IMMOBILISIERUNG EINER AUSWECHSELBAREN KASSETTE IN EINEM GEHÄUSE
SYSTEM FOR IMMOBILISING A REMOVABLE CARTRIDGE IN A HOUSING

(30) Priorité: 28.01.2015 FR 1550668
(43) Date de publication de la demande: 10.01.2018
(73) Titulaire: Valeo Systemes Thermiques, 78320 Le Mesnil Saint Denis (FR)
(72) Inventeur: QUEINNEC, Jean-Yves, 78322 Le Mesnil Saint-Denis Cedex (FR); LECHAT, Yvan, 78322 Le Mesnil Saint-Denis Cedex (FR)
(74) Mandataire: Valeo Systèmes Thermiques
(86) Numéro de dépôt international: PCT/EP2016/051106
(87) Numéro de publication internationale: WO 2016/120137

(56) Documents cités:
- EP-A1- 0 744 309
- EP-A2- 0 903 239
- DE-A1-102008 031 156
- JP-A- H05 162 335
- US-A- 5 155 514

## Description

La présente invention concerne un système d'immobilisation d'une cartouche amovible dans un boîtier, en particulier destiné à être utilisé dans un dispositif de diffusion faisant partie d'un véhicule automobile.

Il est connu des dispositifs comprenant un boîtier et pourvus d'une cartouche amovible, et d'un bouton de verrouillage dudit élément amovible par rapport au boîtier.

C'est le cas des dispositifs de diffusion que l'on trouve dans certains véhicules automobiles, comme décrit dans le document EP 0 903 239 A2. Des agents volatils sont logés dans la cartouche qu'il est nécessaire de changer lorsque les agents volatils sont épuisés. Le bouton de verrouillage/déverrouillage placé sur le boîtier est accessible au passager. Il permet l'enlèvement de la cartouche. Cependant, les mécanismes de blocage de la cartouche activés par ledit bouton restent trop complexes et présentent des risques en termes de fiabilité.

L'invention a pour but d'améliorer la situation et propose à cette fin un ensemble comprenant un système d'immobilisation d'une cartouche amovible dans un boîtier et ladite cartouche amovible, suivant les caractéristiques de la revendication 1. Le système d'immobilisation de la cartouche selon l'invention comprend déclencheur, permettant dans une première position, le maintien de ladite cartouche dans ledit boîtier, et dans une seconde position, l'introduction/extraction de ladite cartouche par rapport audit boîtier, ledit déclencheur étant actionnable en translation selon une direction transversale à une direction d'introduction/extraction de la cartouche.

Une telle orientation du mouvement du déclencheur permet de disposer d'une solution d'immobilisation de la cartouche qui soit simple et robuste. De plus, selon l'invention, ledit déclencheur comprend une barrette de blocage de la cartouche, ladite barrette comprenant au moins une butée de blocage de la cartouche, la ou lesdites butées étant destinées à coopérer avec un ou des pions de blocage de ladite cartouche, le ou lesdits pions de blocage étant munis d'un pan coupé destiné à provoquer un actionnement en translation de la barrette lors de l'introduction de la cartouche.

Selon des caractéristiques complémentaires de l'invention qui pourront être prises ensemble ou séparément :
- ledit déclencheur comprend une languette d'actionnement de la barrette,
- la ou lesdites butées sont situées au niveau d'un contour de ladite barrette,
- ledit déclencheur comprend un élément de rappel, apte à maintenir la barrette dans ladite première position en absence d'actionnement du déclencheur,
- ledit élément de rappel comprend un ressort,
- ledit ressort est configuré pour prendre appui sur ledit boîtier et sur ladite barrette,
- ladite barrette comprend une platine muni d'un doigt de guidage du ressort,
- ledit doigt de guidage est orienté selon la direction de translation de la barrette,
- ladite languette est configurée pour déboucher à travers une façade d'accueil dudit boîtier,
- la cartouche amovible est pourvue d'au moins une cavité,
- la cartouche comprend une pluralité d'ouvertures, en particulier en communication avec la ou lesdites dites cavités,
- lesdites cavités logent au moins un agent volatil,
- les agents volatils comprennent des billes odorantes,
- la cartouche comprend une façade d'introduction dans ledit boîtier,
- les différentes cavités de la cartouche forment des portions angulaires d'un anneau,
- ladite cartouche comprend un corps et un couvercle,
- ledit corps est compartimenté pour définir lesdites cavités,
- le ou lesdits pions de blocage sont situés au niveau d'une face, dite supérieure, de ladite cartouche,
- ladite barrette est mobile en translation le long de ladite face supérieure de la cartouche.

L'invention concerne aussi un dispositif de diffusion comprenant un ensemble tel que décrit plus haut.

Selon différents modes de réalisation de l'invention qui pourront être pris ensemble ou séparément :
- ledit dispositif comprend un organe mobile, dit sélecteur, configuré pour laisser ouverte l'une au moins des ouvertures de la cartouche,
- ledit sélecteur comprend au moins une entrée et une sortie d'un flux d'air, ledit dispositif de diffusion étant configuré pour mettre en communication ladite entrée et ladite sortie, avec l'une au moins desdites cavités pour que cette dernière soit traversée par ledit flux d'air.

L'invention sera mieux comprise à la lumière de la description suivante qui n'est donnée qu'à titre indicatif et qui n'a pas pour but de la limiter, accompagnée des dessins joints parmi lesquels :
- la figure 1 illustre une vue globale en perspective d'un dispositif de diffusion selon l'invention, sans son système d'immobilisation,
- la figure 2 illustre le dispositif de la figure 1, sans son boîtier,
- la figure 3 illustre une vue en perspective de la cartouche du dispositif de la figure 1,
- les figures 4a à 4e illustrent de façon schématique, en vue de dessus, les différentes positions de l'organe mobile servant de sélecteur du dispositif de diffusion de la figure 1,
- la figure 5 illustre en perspective le dispositif de diffusion de la figure 1 équipé de son système d'immobilisation,
- la figure 6 illustre en perspective la cartouche et un élément principal dudit système d'immobilisation
- la figure 7 illustre en perspective le dispositif de diffusion de la figure 1, selon une coupe transversale.

Comme illustré sur les figures, l'invention concerne un système d'immobilisation d'une cartouche amovible dans un boîtier, en particulier pour un dispositif de diffusion permettant le traitement d'un flux d'air à l'aide d'agents volatils, notamment de fragrances.

Selon un mode de réalisation avantageux dudit dispositif, les agents volatils, notamment les fragrances, comprennent un substrat, notamment des billes, chargé en composé(s) volatil(s). Il pourra en particulier s'agir de billes odorantes.

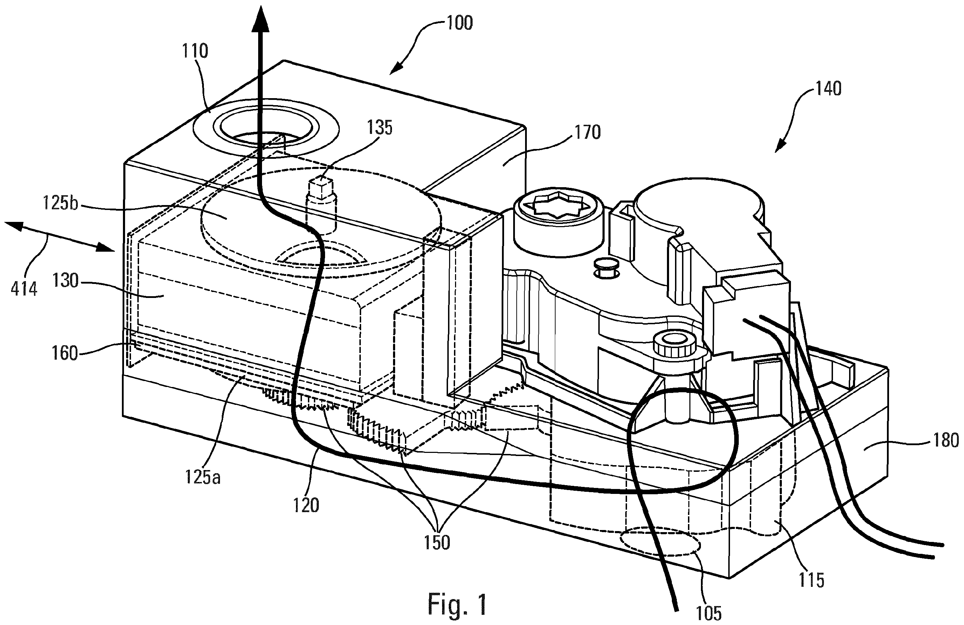

A la figure 1, le dispositif de diffusion ou diffuseur est illustré dans son ensemble. Il comprend ici un boîtier 100 sur lequel deux orifices servent d'entrée 105 et de sortie 110 de l'air. Dès lors, le boîtier sert de guide d'air et comprend une pluralité de composants qui vont permettre à l'air de circuler dans le boîtier. De préférence, une interface sur les orifices 105, 110 permet à une tubulure, non représentée, de s'y fixer tout en assurant l'étanchéité du système à l'air.

Le diffuseur comprend une cartouche 130 de fragrances et un sélecteur, ici logés dans ledit boîtier 100. Le sélecteur comprend, par exemple, un sélecteur d'entrée 125a, un sélecteur de sortie 125b et un axe mobile 135 les entraînant. Ledit diffuseur comprend en outre ici un mécanisme d'actionnement 150, permettant un entraînement dudit axe 135.

Le boîtier 100 peut être fixé à différents emplacements du véhicule, de préférence sur la planche de bord ou dans la boîte à gants afin que ce dernier soit facilement accessible au passager. En effet, la cartouche est amovible par rapport audit boîtier et il est avantageux que le passager puisse facilement changer la cartouche.

La sortie d'air du boîtier 110 est reliée, par exemple, à une grille par laquelle l'air traité ou parfumé sort. Cette grille peut être fixée à différents emplacements dans le véhicule. De préférence, elle est située sur la planche de bord ou près des systèmes de ventilation du pare-brise, la grille étant reliée à la sortie du boîtier par la tubulure déjà évoquée.

Ici, le boîtier 100 comprend une base 180, par exemple parallélépipède rectangle. Dans la base sont logés un pulseur 115 situé au-dessus de l'entrée 105 du boîtier et au moins une partie du mécanisme d'actionnement 150. Ledit mécanisme d'actionnement pourra comprendre un moteur 140. Ce dernier est fixé sur le boîtier 100 au-dessus du pulseur 115, et se trouve en dehors du boîtier. Au-dessus de la base 180, et à côté du moteur 140, une seconde partie du boîtier 170 possède une forme sensiblement cubique pour y loger le sélecteur de fragrances et la cartouche 130. Ces parties du boîtier sont assemblées l'une à l'autre. L'étanchéité à l'air est assurée dans l'ensemble du boîtier. L'orifice d'entrée 105 est donc dans la base 180 du boîtier 100, sur la face inférieure et en dessous du pulseur 115. L'orifice de sortie 110 est sur la face supérieure de la deuxième partie 170, au-dessus du sélecteur de sortie 125b.

L'air pénètre dans le boîtier par l'orifice d'entrée 105, en étant aspiré par le pulseur 115. Ce pulseur génère le flux d'air qui va finalement sortir du boîtier par l'orifice de sortie 110 après avoir traversé la cartouche 130. Le flux d'air est illustré par la ligne 120 sur la figure 1. De préférence, le pulseur 115 est de type radial, mais il peut également être de type axial.

La cartouche 130 est avantageusement composée d'une pluralité de cavités dans lesquelles différentes fragrances peuvent être logées. Le sélecteur 125a, 125b est configuré de sorte que l'une ou plusieurs de ces cavités soient traversées par le flux d'air afin que celui-ci soit chargé des composés volatils de la ou des fragrances sélectionnées. Autrement dit, lorsque le flux d'air traverse la ou lesdites cavités sélectionnées, l'air vient en contact avec l'ensemble du substrat contenu dans chacune desdites cavités et ledit substrat libère le ou les composés qui vont parfumer et/ou traiter le flux d'air, et ensuite l'habitacle du véhicule automobile.

Pour cela, le flux d'air 120 pénètre dans la cartouche 130 par une entrée du sélecteur d'entrée. Le flux d'air traverse alors la cartouche 130 de part en part en passant par l'une ou plusieurs des cavités de ladite cartouche qu'il quitte à travers une sortie dudit sélecteur de sortie 125b. En faisant traverser la ou les cavités sélectionnées par le flux d'air, on s'assure d'un chargement optimisé du flux d'air par la fragrance, celui-ci venant au contact de l'ensemble de la surface du substrat associé. Après être sortie de la cartouche, le flux d'air est ici guidé jusqu'à l'orifice de sortie 110 du boîtier.

De manière préférentielle, le pulseur 115 permet au dispositif d'être indépendant du système de ventilation, de climatisation ou de chauffage dont le véhicule est éventuellement équipé. Il peut dès lors être utilisé indépendamment de ces systèmes, ce qui le rend d'autant plus flexible à l'utilisation. Dans un autre mode de réalisation, il est relié au système de ventilation, de climatisation ou de chauffage du véhicule. L'orifice d'entrée 105 et de sortie 110 seraient dans ce mode de réalisation relié à un conduit dudit système.

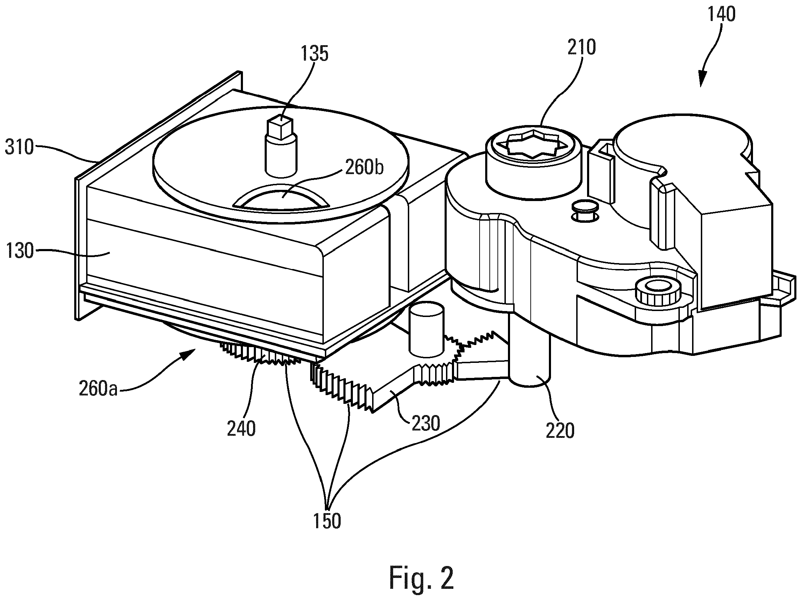

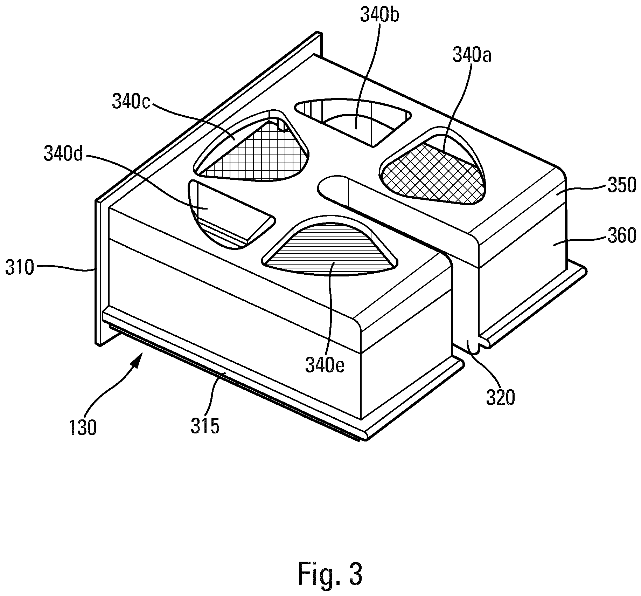

La figure 2 offre une vue plus détaillée de la figure 1 sans le boîtier 100. La figure 2 illustre les détails du mécanisme d'actionnement 150, de l'axe mobile 135 et du sélecteur d'entrée 125a et de sortie 125b. La cartouche 130 est illustrée plus en détails sur la figure 3.

Le mécanisme d'actionnement 150 comprend par exemple le moteur 140, un arbre 220, un pignon de transmission 230, permettant avantageusement une démultiplication, et un pignon droit 240. Le moteur 140 entraîne la cinématique des éléments situés en aval : l'arbre 220, directement relié à un actuateur du moteur, entraîne le pignon de transmission 230, qui entraîne le pignon droit 240. L'axe mobile 135 est directement fixé sur le pignon droit 240 et est dès lors également entraîné par ce dernier.

Avantageusement, le sélecteur d'entrée 125a et le sélecteur de sortie 125b se présentent sous la forme de plateaux tournants, l'axe mobile 135 perçant les deux plateaux tournants en leur centre. La forme du trou perçant les plateaux tournants correspond à la forme d'une section de l'axe mobile, de préférence de forme carrée de telle sorte que lorsque l'axe mobile tourne, les plateaux tournants tournent également. Les plateaux tournants sont séparés par une distance correspondant à la hauteur de la cartouche 130 afin de permettre à la cartouche d'y être insérée. Lorsque la cartouche est insérée entre les deux plateaux, ces derniers sont avantageusement pressés contre la cartouche par des ressorts, non illustrés, afin d'assurer l'étanchéité à l'air.

De préférence, l'entrée et la sortie du flux d'air sur les plateaux tournants situés respectivement en amont et en aval de la cartouche se présentent sous la forme d'ouvertures débouchantes 260b (l'ouverture 260a en amont de la cartouche n'est pas visible) dont la taille est comparable à celle d'une entrée, respectivement d'une sortie d'air, d'une cavité de la cartouche. Les ouvertures des deux plateaux tournants sont disposées en vis-à-vis de manière à assurer la continuité du flux d'air. D'autre part, les positions des ouvertures desdits plateaux tournants restent synchronisées pendant leur mouvement grâce à l'axe mobile 135 qui les relie.

La figure 3 offre une vue de la cartouche isolée 130. Comme déjà dit, la cartouche 130 est un élément amovible par rapport au boîtier 100. Le passager du véhicule peut alors facilement changer de cartouche, notamment lorsqu'elle est vide, la durée de vie d'une cartouche étant d'environ 3 mois pour une utilisation normale. Par « vide », on entend dont les fragrances sont épuisées et ne libèrent plus de composés volatils.

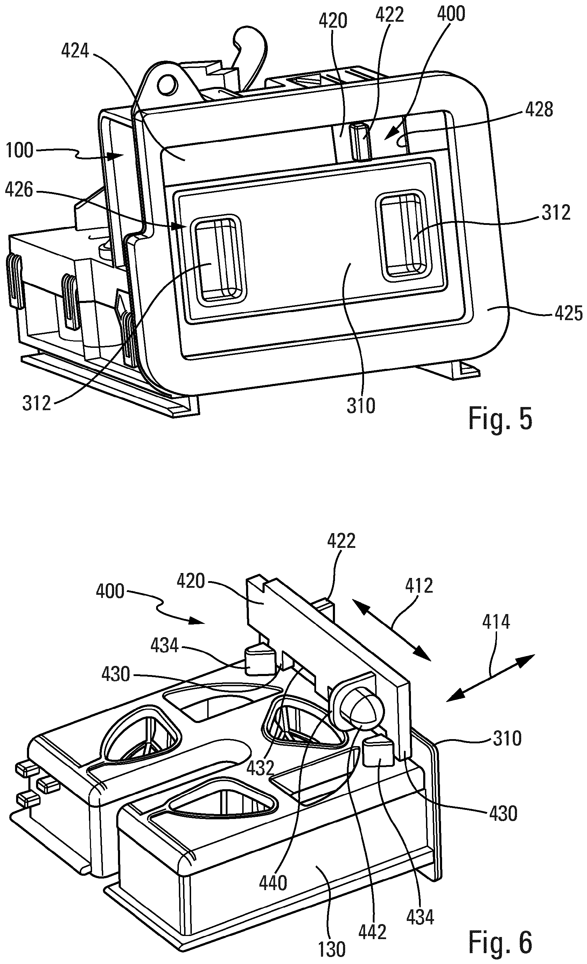

La cartouche est ici munie d'une façade d'introduction 310 dans ledit boîtier, se présentant de manière similaire à la façade d'un tiroir. Ladite façade 310 est ici munie de logements 312 (figure 5) facilitant l'insertion de ladite cartouche 130 dans le boîtier 100. La cartouche 130 est également pourvue d'une nervure longitudinale 315 de guidage située le long des trois faces verticales qui vont s'insérer dans le boîtier 100. Pour accueillir lesdites nervures de guidage 315 de la cartouche 130, le boîtier possède également des rainures de guidage 160, illustrées sur la figure 1, de part et d'autre du logement de la cartouche dans le boîtier et au-dessus du plateau tournant inférieur 125a.

Une fente 320 permet le passage de l'axe lorsque la cartouche est insérée dans le boîtier entre les deux plateaux tournants 125a et 125b illustrés aux figures 1 et 2. En position d'utilisation, l'axe mobile 135 se trouve au centre de la cartouche dans le fond de la fente.

La cartouche comprend un corps 360 et un couvercle 350 dont la forme est parallélépipédique et la base est sensiblement carrée. Le corps est compartimenté pour définir lesdites cavités, le fond dudit corps comprend les entrées du flux d'air desdites cavités, et le couvercle comprend les sorties du flux d'air desdites cavités. La cartouche est de préférence en matière plastique. Lesdites ouvertures pourront être revêtues d'un grillage ou d'un film poreux pour retenir le substrat desdites fragrances tout en laissant passer le flux d'air à travers.

Avantageusement, des cavités occupées par une fragrance et des cavités vides alternent. L'alternance de cavités vides et de cavités logeant une fragrance permet de passer de chacune des cavités comprenant un fragrance à une cavité n'en comportant pas, sans passer par un cavité comprenant une autre fragrance.

Dans un mode de réalisation tel qu'illustré sur la figure 3, les différentes cavités de la cartouche et leurs ouvertures respectives forment des portions angulaires d'un anneau. Dans ce mode de réalisation particulièrement avantageux, cinq cavités sont prévues, dont trois pour loger une fragrance 340a, 340c, 340e et deux sont laissées vides intentionnellement 340b, 340d. L'angle balayé par les ouvertures de chaque portion angulaire est dans ce mode de réalisation légèrement inférieur à 60 degrés. Dans un tel mode de réalisation, les ouvertures 260a, 260b des plateaux se présentent également sous la forme d'une portion angulaire, chaque plateau comprenant ici une unique ouverture 260a, respectivement 260b.

Le passager peut dès lors choisir entre trois fragrances différentes selon ses goûts : différentes ambiances sont envisageables pendant le voyage, ce qui peut être particulièrement agréable pour les longs voyages. Ces différentes fragrances peuvent également être très utiles pour masquer ou filtrer une odeur indésirable de tabac par exemple. Le passager peut également choisir de laisser le système de ventilation en marche sans pour autant être en présence d'une fragrance.

Une telle distribution des cavités, sous la forme de portions angulaires en anneau, permet de facilement augmenter le nombre de cavités sans pour autant devoir modifier le mécanisme d'actionnement associé. Le seul changement à effectuer pour augmenter le nombre de fragrances disponibles au passager réside dans la taille des cavités de la cartouche et dans la taille des ouvertures des plateaux tournants qui leur correspondent.

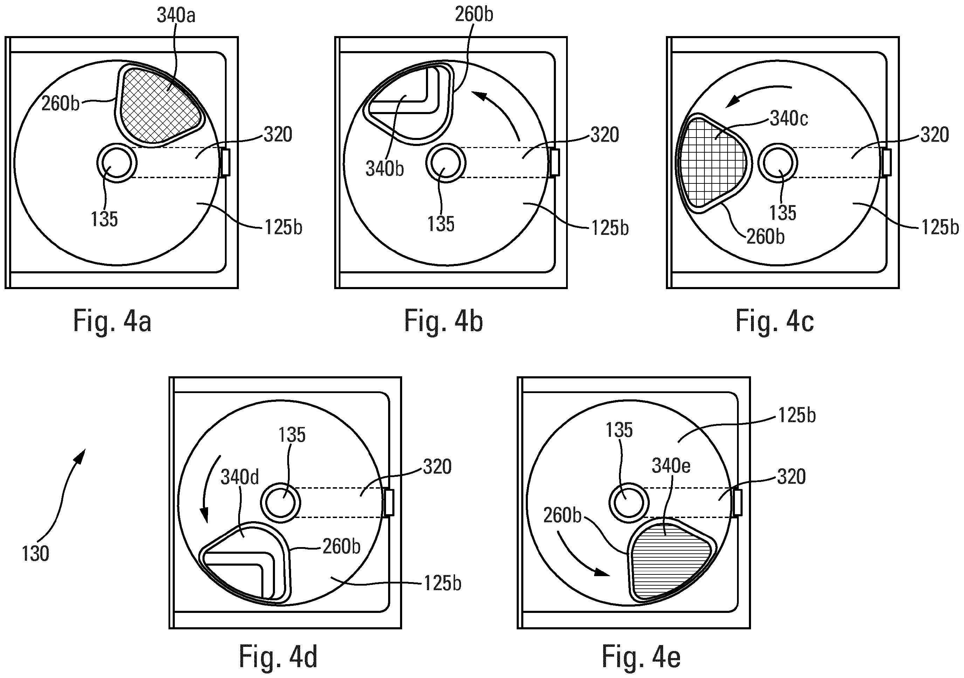

Les figure 4a à 4b illustrent une vue du haut du mécanisme permettant le changement de fragrances effectué par l'ensemble comprenant la cartouche 130 et le sélecteur 125a, 125b. La figure ne montre que le plateau tournant 125b situé sur la cartouche 130. Cependant, la vue du bas serait représentée de la même manière. L'axe mobile 135 ainsi que la fente 320 de la cartouche 130 sont également visibles sur la figure.

Dans le mode de réalisation décrit à la figure 3, où la cartouche comprend cinq cavités dont trois sont dédiées à loger une fragrance et deux sont laissées vides, les figures 4a à 4e illustrent le passage de l'ouverture 260b de la plaque tournante 125b sur chacune des cinq positions. La figure 4a illustre la première position de la plaque tournante où la fragrance libérée provient de la cavité 340a de la figure 3. Pour passer à la deuxième position, illustrée sur la figure 4b, la plaque tournante va tourner dans le sens antihoraire d'un angle d'environ 60 degrés. Cette position correspond à une cavité vide de la cartouche correspondant à la cavité 340b de la figure 3. La figure 4c illustre l'effet d'une rotation supplémentaire dans le sens antihoraire d'environ 60 degrés par rapport à la deuxième position permettant de libérer la deuxième fragrance logée dans la cavité 340c. De la même manière, les figures 4d et 4e illustrent l'ouverture du plateau tournant située respectivement sur les cavités 340d pour le quatrième position et 340e pour la cinquième position de la cartouche.

Préférentiellement, pour passer à une position ultérieure, la plaque tournante va tourner dans le sens horaire. En effet, il y a une zone par laquelle la plaque tournante ne passe pas pendant son utilisation. Cette zone se trouve au-dessus de la fente 320, ou entre les cavités 340a et 340e.

L'alternance de cavités vides et de cavités logeant une fragrance permet au passager de régler l'intensité de la fragrance diffusée par un actionnement du sélecteur en va-et-vient. Dans le même but, il est également possible de placer l'ouverture du plateau tournant en une position intermédiaire entre la cavité vide et la cavité remplie. Lorsque l'ouverture recouvre en totalité l'ouverture de la cavité, alors la diffusion est totale, lorsque l'ouverture recouvre seulement une partie de l'ouverture de la cavité comportant l'agent volatil, alors la diffusion n'est que partielle.

La vitesse du plateau tournant pour passer d'une fragrance à une utilisation sans fragrance est avantageusement de 0.5 seconde. Par suite, la vitesse de transition pour passer de la première position à la cinquième position, et vice-versa, est de 2.5 secondes.

Ce changement rapide entre différentes positions est rendu possible grâce au mécanisme d'actionnement 150 illustré sur les figures 1 et 2, et déjà décrit en partie.

Dans un mode de réalisation préféré, le moteur 140 est de type moto-réducteur pas à pas. Ce type de moteur standard est choisi pour des raisons de coût et de disponibilité sur le marché. Comme illustré sur les figures 1 et 2, l'arbre et le pignon de transmission sont à portion angulaire. En effet, la plage de rotation des plateaux tournants n'est pas de 360°, mais de 240° comme discuté ci-dessus.

C'est la combinaison de l'arbre 220, du pignon de transmission 230 et du pignon droit 240 qui permet une démultiplication du mouvement induit par le moto-réducteur 140 de telle sorte que le sélecteur passe d'une position à la suivante en 0.5 secondes et de la première position à la cinquième position en 2.5 secondes, et vice-versa. La combinaison de ces trois éléments forme un organe de démultiplication qui permet d'obtenir de telles performances avec un moteur standard.

Un système de commande du dispositif pourra comprendre un module de programmation et l'électronique associée afin de permettre au passager de contrôler le pulseur et le moteur du mécanisme d'actionnement électrique. Préférentiellement, le passager peut contrôler le distributeur de fragrances à partir d'une commande au volant.

Le système de commande permet au conducteur de mettre le distributeur de fragrances sous tension, de choisir l'intensité de la fragrance diffusée, de choisir le débit du flux d'air diffusé et/ou de choisir la fragrance. Avantageusement, le système de commande indique lorsqu'une fragrance de la cartouche est vide.

Dans un premier temps, le système de commande permet au passager de choisir la fragrance qu'il souhaite diffuser. Ensuite, il peut également régler le débit du flux d'air généré par le pulseur. Le système de commande permet également au passager de régler l'intensité de la fragrance qu'il souhaite diffuser.

Le système de commande pourra en outre permettre au passager de préprogrammer une séquence de diffusion qu'il souhaite en choisissant une durée de diffusion, une fragrance et son intensité. L'intensité pourra également varier au cours du temps ; il est dès lors possible de commencer par une diffusion totale qui devient partielle au cours du temps. Le système de commande permet également de choisir une fréquence de diffusion. Par exemple, le passager peut choisir de diffuser une certaine fragrance pendant une durée choisie, et puis ne rien diffuser pendant une autre durée.

Comme illustré aux figures 5 à 6, le système d'immobilisation de la cartouche 130 comprend un déclencheur 400, permettant dans une première position, le maintien de ladite cartouche dans ledit boîtier, et dans une seconde position, l'introduction/extraction de ladite cartouche 130 par rapport audit boîtier 100. A la figure 6, le déclencheur 400 est dans sa première position.

Selon l'invention, ledit déclencheur 400 est actionnable en translation selon une direction, illustrée par une flèche repérée 412 à la figure 6, transversale, en particulier orthogonale, à une direction d'introduction/extraction de la cartouche, illustrée par une flèche repérée 414 à la figure 6 (ainsi qu'à la figure 1). Une telle orientation permet de diposer d'un déclencheur simple et robuste.

Ledit déclencheur 400 comprend, selon l'invention, une barrette 420 de blocage de la cartouche 130 et comprend par exemple une languette 422 d'actionnement de la barrette 420. Ladite languette 422 fait ici saillie perpendiculairement à la barette avec laquelle elle est éventuellement issue de matière.

Le boîtier 100 comprend une face frontale 424, munie d'un orifice 426 d'introduction/extraction de la cartouche 130. Ladite face frontale 424 est également munie d'une encoche 428 de passage de la languette 422, permettant à l'utilisateur de manipuler cette dernière depuis l'extérieur, selon un mouvement orienté suivant la direction 412. Ladite encoche 428 pourra être formée dans le contour de l'orifice 426 d'introduction/extraction de la cartouche 130 ou, comme ici, être indépendante. Avec un cadre 425 du dispositif, ladite face frontale 424 du boîtier forme une façade d'accueil de la cartouche 130.

Ladite languette 422 pourra être configurée pour venir en vis-à-vis de de deux bords longitudinaux opposés de ladite encoche 428, avec un léger jeu, de sorte que ladite languette 422 est guidée le long desdits bord longitudinaux de l'encoche, facilitant ainsi le guidage de la barrette 420 dans son mouvement de translation.

Ladite barrette 420 comprend, selon l'invention, au moins une butée 430, ici deux butées 430, de blocage de la cartouche 130. Dans l'exemple, lesdites butées 430 sont situées au niveau d'un contour de ladite barrette 420, notamment le long de l'un de ses bords 432, dit inférieur, au niveau de deux extrémités longitudinales opposées de ladite barrette 420.

De son côté, ladite cartouche comprend au moins un pion de blocage 434, ici deux pions de blocage 434, destiné chacun à coopérer avec l'une desdites butées 430. Dans l'exemple, ladite barrette 420 est mobile en translation le long d'une face de la cartouche, notamment la face supérieure, à savoir la face de la cartouche au niveau de laquelle se déplace ledit plateau tournant supérieur 125b. Lesdits pions de blocage 434 sont également situés au niveau de ladite face supérieure, au niveau de sommets de la cartouche de façon à être en dehors de l'encombrement dudit plateau tournant supérieur 125b, lorsque la cartouche est en position.

De façon préférentielle, lesdit pions de blocage 434 sont légèrement en retrait d'une face arrière de la façade 310 de la cartouche 130 de sorte que ladite barette 420 est guidée dans son mouvement de translation entre lesdits pions 434 et ladite façade 310.

Le ou lesdits pions de blocage 434 sont avantageusement munis d'un pan coupé, destiné à provoquer un actionnement en translation de la barrette 420 lors de l'introduction de la cartouche 130.

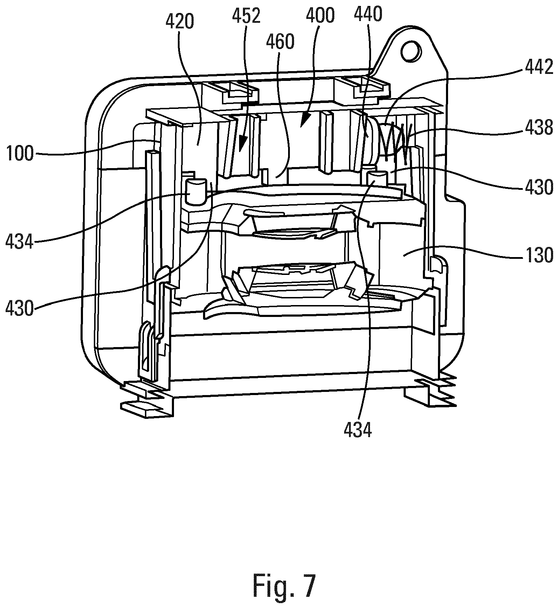

Comme illustré à la figure 7, ledit déclencheur 400 pourra en outre comprendre un élément de rappel, tel qu'un ressort 438, apte à maintenir la barrette 420 dans ladite première position en absence d'actionnement du déclencheur 400, c'est-à-dire, tant que la languette 422 n'est pas déplacée par un utilisateur souhaitant retirer la cartouche 130. Ledit ressort 438 prend appui sur le boîtier 100, ici sur une face latérale dudit boîtier, et sur ladite barrette 420, par exemple par l'intermédiaire d'une platine 440 muni d'un doigt de guidage 442 du ressort 438. Ladite platine 440 pourra être issue de ladite barrette 420. Ledit doigt de guidage 442 est avantageusement orienté selon la direction de translation 412 de la barrette 420.

A la figure 7, on constate que ledit boîtier 100 pourra encore comprend un ou des plots 452 de guidage de la barrette 420, ici issus de la face supérieure dudit boîtier. Ladite barrette 420 est située entre lesdits plots de guidage 452 et ladite face frontale du boîtier 100.

Toujours à la figure 7, on constate que lesdites encoches 430 sont distantes l'une de l'autre le long de ladite barrette 420. Une encoche intermédiaire 460 est ici prévue le long dudit bord inférieur 432 de la barrette, entre lesdites encoches 430 coopérant avec les butées 434.

Bien que le système d'immobilisation conforme à l'invention ait été décrit dans le cadre d'un dispositif de diffusion de plusieurs fragrances, il pourra aussi être utilisé avec des cartouches ne servant qu'à une seule fragrance. Il pourra aussi être utilisé avec des cartouches servant à une autre application qu'un système de diffusion.

## Revendications

1. Ensemble comprenant un système d'immobilisation d'une cartouche amovible (130) dans un boîtier (100) et ladite cartouche amovible (130), ledit système comprenant un déclencheur (400), permettant dans une première position, le maintien de ladite cartouche (130) dans ledit boîtier (100), et dans une seconde position, l'introduction/extraction de ladite cartouche (130) par rapport audit boîtier, **caractérisé en ce que** ledit déclencheur (400) est actionnable en translation selon une direction transversale à une direction d'introduction/extraction de la cartouche (130), ledit déclencheur (400) comprenant une barrette (420) de blocage de la cartouche (130), ladite barrette (420) comprenant au moins une butée (430) de blocage de la cartouche (130), la ou lesdites butées (430) étant destinées à coopérer avec un ou des pions de blocage (434) de ladite cartouche (130), ensemble dans lequel le ou lesdits pions de blocage (434) sont munis d'un pan coupé destiné à provoquer un actionnement en translation de la barrette (420) lors de l'introduction de la cartouche (130).

2. Ensemble selon la revendication 1 dans lequel ledit déclencheur (400) comprend une languette (422) d'actionnement de la barrette (420).

3. Ensemble selon la revendication 2 dans lequel ladite languette (422) est configurée pour déboucher à travers une façade d'accueil dudit boîtier (100).

4. Ensemble selon la revendication 1 dans lequel la ou lesdites butées (430) sont situées au niveau d'un contour de ladite barrette (420).

5. Ensemble selon l'une quelconque des revendications précédentes dans lequel ledit déclencheur (400) comprend un élément de rappel, apte à maintenir la barrette (420) dans ladite première position en absence d'actionnement du déclencheur.

6. Ensemble selon la revendication 5 dans lequel ledit élément de rappel comprend un ressort (438), ledit ressort étant configuré pour prendre appui sur ledit boîtier (100) et sur ladite barrette (420).

7. Ensemble selon la revendication 6 dans lequel ladite barrette (420) comprend une platine (440) muni d'un doigt (442) de guidage du ressort (438).

8. Ensemble selon l'une quelconque des revendications précédentes dans lequel le ou lesdits pions de blocage (434) sont situés au niveau d'une face, dite supérieure, de ladite cartouche (130).

9. Ensemble selon la revendication 8 dans lequel ladite barrette (420) est mobile en translation le long de ladite face supérieure de la cartouche (130).

10. Dispositif de diffusion d'agent volatil comprenant l'ensemble selon l'une quelconque des revendications 1 à 9.

## Patentansprüche

1. Anordnung, umfassend ein System zur Immobilisierung einer entnehmbaren Patrone (130) in einem Gehäuse (100) und die entnehmbare Patrone (130), wobei das System einen Auslöser (400) umfasst, der in einer ersten Stellung das Halten der Patrone (130) in dem Gehäuse (100) und in einer zweiten Stellung das Einführen/Entnehmen der Patrone (130) in Bezug auf das Gehäuse ermöglicht, **dadurch gekennzeichnet, dass** der Auslöser (400) translatorisch entlang einer Richtung quer zu einer Einführ-/Entnahmerichtung der Patrone (130) betätigbar ist, wobei der Auslöser (400) einen Schieber (420) zum Sichern der Patrone (130) umfasst, wobei der Schieber (420) mindestens einen Anschlag (430) zum Sichern der Patrone (130) umfasst, wobei der oder die Anschläge (430) dazu bestimmt sind, mit einem oder mehreren Sicherungsstiften (434) der Patrone (130) zusammenzuwirken, wobei bei der Anordnung der oder die Sicherungsstifte (434) mit einer Schräge versehen sind, die dazu bestimmt ist, beim Einführen der Patrone (130) eine translatorische Betätigung des Schiebers (420) zu bewirken.

2. Anordnung nach Anspruch 1, wobei der Auslöser (400) einen Betätigungslappen (422) des Schiebers (420) umfasst.

3. Anordnung nach Anspruch 2, bei welcher der Lappen (422) dazu ausgestaltet ist, durch eine Aufnahmefront des Gehäuses (100) hindurch zu münden.

4. Anordnung nach Anspruch 1, bei welcher der oder die Anschläge (430) an einer Kontur des Schiebers (420) gelegen sind.

5. Anordnung nach einem der vorhergehenden Ansprüche, bei welcher der Auslöser (400) ein Rückstellelement umfasst, das geeignet ist, den Schieber (420) bei Nichtbetätigung des Auslösers in der ersten Stellung zu halten.

6. Anordnung nach Anspruch 5, bei der das Rückstellelement eine Feder (438) umfasst, wobei die Feder dazu ausgestaltet ist, an dem Gehäuse (100) und an dem Schieber (420) anzuliegen.

7. Anordnung nach Anspruch 6 wobei der Schieber (420) eine Platte (440) umfasst, die mit einem Finger (442) zum Führen der Feder (438) versehen ist.

8. Anordnung nach einem der vorhergehenden Ansprüche, bei welcher der oder die Sicherungsstifte (434) an einer Seite, Oberseite genannt, der Patrone (130) gelegen sind.

9. Anordnung nach Anspruch 8, bei welcher der Schieber (420) translatorisch entlang der Oberseite der Patrone (130) beweglich ist.

10. Vorrichtung zum Verbreiten eines flüchtigen Stoffs, die eine Anordnung nach einem der Ansprüche 1 bis 9 umfasst.

## Claims

1. Assembly comprising a system for immobilizing a removable cartridge (130) in a housing (100) and said removable cartridge (130), said system comprising a trigger (400) which, in a first position, allows said cartridge (130) to be held in said housing (100) and, in a second position, allows said cartridge (130) to be inserted/extracted with respect to said housing, **characterized in that** said trigger (400) is actuable in translation in a direction transverse to a direction of insertion/extraction of the cartridge (130), said trigger (400) comprising a bar (420) for blocking the cartridge (130), said bar (420) comprising at least one stop (430) for blocking the cartridge (130), said stop(s) (430) being intended to cooperate with one or more blocking studs (434) of said cartridge (130), in which assembly said blocking stud(s) (434) is (are) provided with a bevel intended to cause the bar (420) to be actuated in translation when inserting the cartridge (130).

2. Assembly according to Claim 1, in which said trigger (400) comprises a tab (422) for actuating the bar (420).

3. Assembly according to Claim 2, in which said tab (422) is configured to emerge through a reception façade of said housing (100).

4. Assembly according to Claim 1, in which said stop(s) (430) is (are) situated on a contour of said bar (420).

5. Assembly according to any one of the preceding claims, in which said trigger (400) comprises a return element able to keep the bar (420) in said first position in the absence of actuation of the trigger.

6. Assembly according to Claim 5, in which said return element comprises a spring (438), said spring being configured to bear on said housing (100) and on said bar (420) .

7. Assembly according to Claim 6, in which said bar (420) comprises a plate (440) provided with a finger (442) for guiding the spring (438).

8. Assembly according to any one of the preceding claims, in which said blocking stud(s) (434) is (are) situated on a face, termed upper face, of said cartridge (130) .

9. Assembly according to Claim 8, in which said bar (420) is movable in translation along said upper face of the cartridge (130).

10. Volatile agent diffusion device comprising the assembly according to any one of Claims 1 to 9.
